(19) Europäisches Patentamt
European Patent Office
Office européen des brevets

(11) EP 1 863 488 B1

(12) **EUROPÄISCHE PATENTSCHRIFT**

(45) Veröffentlichungstag und Bekanntmachung des Hinweises auf die Patenterteilung:
**24.02.2010 Patentblatt 2010/08**

(21) Anmeldenummer: **06723256.1**

(22) Anmeldetag: **07.03.2006**

(51) Int Cl.:
***A61K 31/454*** *(2006.01)* ***A61P 3/10*** *(2006.01)*
***C07D 401/04*** *(2006.01)*

(86) Internationale Anmeldenummer:
**PCT/EP2006/002057**

(87) Internationale Veröffentlichungsnummer:
**WO 2006/099942 (28.09.2006 Gazette 2006/39)**

(54) **VERWENDUNG VON AMINO SUBSTITUIERTEN BENZIMIDAZOLEN**
USE OF AMINO-SUBSTITUTED 8-N-BENZIMIDAZOLES
UTILISATION DE 8-N-BENZIMIDAZOLES A SUBSTITUTION AMINO

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.03.2005 DE 102005012875**

(43) Veröffentlichungstag der Anmeldung:
**12.12.2007 Patentblatt 2007/50**

(73) Patentinhaber: **Sanofi-Aventis**
**75013 Paris (FR)**

(72) Erfinder:
- **DEFOSSA, Elisabeth**
  **65926 Frankfurt am Main (DE)**
- **SCHOENAFINGER, Karl**
  **65926 Frankfurt am Main (DE)**
- **JAEHNE, Gerhard**
  **65926 Frankfurt am Main (DE)**
- **BUNING, Christian**
  **65926 Frankfurt am Main (DE)**
- **TSCHANK, Georg**
  **65926 Frankfurt am Main (DE)**
- **WERNER, Ulrich**
  **65926 Frankfurt am Main (DE)**

(74) Vertreter: **Fischer, Hans-Jürgen**
**Sanofi-Aventis Deutschland GmbH**
**Patent- und Lizenzabteilung**
**Industriepark Höchst**
**Gebäude K 801**
**65926 Frankfurt am Main (DE)**

(56) Entgegenhaltungen:
**EP-A1- 1 069 124** **WO-A-03/104233**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von Amino substituierten 8-N-Benzimidazolen sowie derer physiologisch verträglichen Salze zur Herstellung eines Medikaments zur Blutzuckersenkung. Diese Verbindungen sollen sich insbesonders zur Herstellung eines Medikaments zur Behandlung von Diabetes eignen.

EP 1069124 beschreibt 2-Benzimdazolylamine als ORL-1 Rezeptor Agonisten.
WO 97/12615 beschreibt Benzimidazolderivate als 15-LO Inhibitoren.
WO 02/04425 beschreibt strukturähnliche Virus Polymerase Inhibitoren.

Der Erfindung lag die Aufgabe zugrunde, Verbindungen zur Verfügung zu stellen, die eine therapeutisch verwertbare Blutzucker senkende Wirkung entfalten. Diese Verbindungen sollen sich insbesonders zur Behandlung von Diabetes eignen.

Die Erfindung betrifft daher die Verwendung der Verbindungen der Formel I,

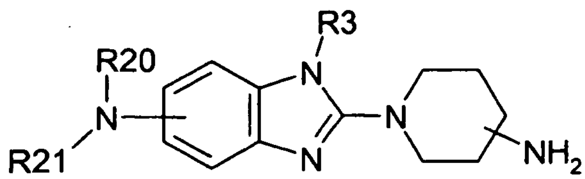

I

worin bedeuten

R20, R21 unabhängig voneinander H, $(C_1-C_6)$-Alkylen-$(C_6-C_{10})$-Aryl oder $S(O)_2$-Aryl, wobei die Aryl-Reste ein- oder mehrfach substituiert sein können mit F, Cl, Br, I, CN, $NO_2$, SH, OH, $(C_1-C_6)$-Alkyl, O-$(C_1-C_6)$-Alkyl oder S-$(C_1-C_6)$-Alkyl;

R3 $(C_2-C_{10})$-Alkenyl, $(C_1-C_6)$-Alkylen-$(C_6-C_{10})$-Aryl;

sowie derer physiologisch verträglichen Salze zur Herstellung eines Medikaments zur Blutzuckersenkung

Die Erfindung bezieht sich auf die Verwendung der Verbindungen der Formel I, in Form ihrer Razemate, razemischen Mischungen und reinen Enantiomeren sowie auf ihre Diastereomeren und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, können als nützliche Zwischenprodukte für die Herstellung oder Reinigung pharmazeutisch verträglicher Salze und/oder für die Verwendung in nicht-therapeutischen, zum Beispiel in-vitro-Anwendungen verwendet werden.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, $CF_3$, $NO_2$, $N_3$, CN, COOH, COO$(C_1-C_6)$Alkyl, $CONH_2$, CONH$(C_1-C_6)$Alkyl, CON$[(C_1-C_6)Alkyl]_2$, Cycloalkyl, $(C_2-C_6)$-Alkenyl,

$(C_2-C_6)$-Alkinyl, O-$(C_1-C_6)$-Alkyl, O-CO-$(C_1-C_6)$-Alkyl, O-CO-$(C_1-C_6)$-Aryl, O-CO-$(C_1-C_6)$-Heterocyclus; $PO_3H_2$, $SO_3H$, $SO_2$-$NH_2$, $SO_2NH(C_1-C_6)$-Alkyl, $SO_2N[(C_1-C_6)$-Alkyl$]_2$, S-$(C_1-C_6)$-Alkyl, S-$(CH_2)_n$-Aryl, S-$(CH_2)_n$-Heterocyclus, SO-$(C_1-C_6)$-Alkyl, SO-$(CH_2)_n$-Aryl, SO-$(CH_2)_n$-Heterocyclus, $SO_2$-$(C_1-C_6)$-Alkyl, $SO_2$-$(CH_2)_n$-Aryl, $SO_2$-$(CH_2)_n$-Heterocyclus, $SO_2$-NH$(CH_2)_n$-Aryl, $SO_2$-NH$(CH_2)_n$-Heterocyclus, $SO_2$-N($(C_1-C_6)$-Alkyl)$(CH_2)_n$Aryl, $SO_2$-N($(C_1-C_6)$-Alkyl) $(CH_2)_n$-Heterocyclus, $SO_2$-N($(CH_2)_n$-Aryl$)_2$, $SO_2$-N($(CH_2)_n$-Heterocyclus$)_2$ wobei n = 0 - 6 sein kann und der Arylrest oder Heterocyclische Rest bis zu zweifach mit F, Cl, Br, OH, $CF_3$, $NO_2$, CN, $OCF_3$, O-$(C_1-C_6)$-Alkyl, $(C_1-C_6)$-Allcyl oder $NH_2$ substituiert sein kann; C(=NH)($NH_2$), $NH_2$, NH-$(C_1-C_6)$-Alkyl, N($(C_1-C_6)$-Alkyl$)_2$, NH-CO-$(C_1-C_6)$-Alkyl, NH-COO-$(C_1-C_6)$-Alkyl, NH-CO-Aryl, NH-CO-Heterocyclus, NH-COO-Aryl, NH-COO-Heterocyclus, NH-CO-NH-$(C_1-C_6)$-Alkyl, NH-CO-NH-Aryl, NH-CO-NH-Heterocyclus, N($(C_1-C_6)$-Alkyl)-CO-$(C_1-C_6)$-Alkyl, N($(C_1-C_6)$-Alkyl)-COO-$(C_1-C_6)$-Alkyl, N($(C_1-C_6)$-Alkyl)-CO-Aryl, N($(C_1-C_6)$-Alkyl)-CO-Heterocyclus, N($(C_1-C_6)$-Alkyl)-COO-Aryl, N($(C_1-C_6)$-Alkyl)-COO-Heterocyclus, N($(C_1-C_6)$-Alkyl)-CO-NH-$(C_1-C_6)$-Alkyl), N($(C_1-C_6)$-Alkyl)-CO-NH-Aryl, N($(C_1-C_6)$-Alkyl)-CO-NH-Heterocyclus, N($(C_1-C_6)$-Alkyl)-CO-N($(C_1-C_6)$-Alkyl$)_2$, N($(C_1-C_6)$-Alkyl)-CO-N($(C_1-C_6)$-Alkyl)-Aryl, N($(C_1-C_6)$-Alkyl)-CO-N($(C_1-C_6)$-Alkyl)-Heterocyclus, N($(C_1-C_6)$-Alkyl)-CO-N-(Aryl$)_2$, N($(C_1-C_6)$-Alkyl)-CO-N-(Heterocyclus$)_2$, N (Aryl)-CO-$(C_1-C_6)$-Alkyl, N(Heterocyclus)-CO-$(C_1-C_6)$-Alkyl, N(Aryl)-COO-$(C_1-C_6)$-Alkyl, N(Heterocyclus)-COO-$(C_1-C_6)$-Alkyl, N(Aryl)-CO-Aryl, N(Heterocyclus)-CO-Aryl, N(Aryl)-COO-Aryl, N(Heterocyclus)-COO-Aryl, N (Aryl)-CO-NH-$(C_1-C_6)$-Alkyl), N(Heterocyclus)-CO-NH-$(C_1-C_6)$-Alkyl), N(Aryl)-CO-NH-Aryl, N(Heterocyclus)-CO-NH-Aryl, N(Aryl)-CO-N($(C_1-C_6)$-Alkyl$)_2$, N(Heterocyclus)-CO-N($(C_1-C_6)$-Alkyl$)_2$, N(Aryl)-CO-N($(C_1-C_6)$-Alkyl)-Aryl, N(Heterocyclus)-CO-N($(C_1-C_6)$-Alkyl)-Aryl, N(Aryl)-CO-N-(Aryl$)_2$, N(Heterocyclus)-CO-N-(Aryl$)_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-$(C_1-C_6)$-Alkyl $(C_1-C_6)$-Alkyl, $NH_2$, NH$(C_1-C_6)$-Alkyl N($(C_1-C_6)$-Alkyl$)_2$, $SO_2$-$CH_3$, COOH, COO-$(C_1-C_6)$-Alkyl oder $CONH_2$.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl, 2-Methyl-but-2-en-4-yl.

Die Alkenylreste können ein oder mehrfach mit geeigneten Gruppen substituiert sein, wie z.B.: F, Cl, Br, I, $CF_3$, $NO_2$, $N_3$, CN, COOH, COO$(C_1-C_6)$Alkyl, $CONH_2$, CONH$(C_1-C_6)$Alkyl, CON[$(C_1-C_6)$Alkyl$]_2$, Cycloalkyl, $(C_2-C_6)$-Alkenyl, $(C_2-C_6)$-Alkinyl, O-$(C_1-C_6)$-Alkyl, O-CO-$(C_1-C_6)$-Alkyl, O-CO-$(C_1-C_6)$-Aryl, O-CO-$(C_1-C_6)$-Heterocyclus; $PO_3H_2$, $SO_3H$, $SO_2$-$NH_2$, $SO_2NH(C_1-C_6)$-Alkyl, $SO_2N[(C_1-C_6)$-Alkyl$]_2$, S-$(C_1-C_6)$-Alkyl, S-$(CH_2)_n$-Aryl, S-$(CH_2)_n$-Heterocyclus, SO-$(C_1-C_6)$-Alkyl, SO-$(CH_2)_n$-Aryl, SO-$(CH_2)_n$-Heterocyclus, $SO_2$-$(C_1-C_6)$-Alkyl, $SO_2$-$(CH_2)_n$-Aryl, $SO_2$-$(CH_2)_n$-Heterocyclus, $SO_2$-NH$(CH_2)_n$-Aryl, $SO_2$-NH$(CH_2)_n$-Heterocyclus, $SO_2$-N($(C_1-C_6)$-Alkyl)$(CH_2)_n$-Aryl, $SO_2$-N($(C_1-C_6)$-Alkyl) $(CH_2)_n$-Heterocyclus, $SO_2$-N($(CH_2)_n$-Aryl$)_2$, $SO_2$-N($(CH_2)_n$-Heterocyclus$)_2$ wobei n = 0 - 6 sein kann und der Arylrest oder Heterocyclische Rest bis zu zweifach mit F, Cl, Br, OH, $CF_3$, $NO_2$, CN, $OCF_3$, O-$(C_1-C_6)$-Alkyl, $(C_1-C_6)$-Alkyl oder $NH_2$ substituiert sein kann; C(=NH)($NH_2$), $NH_2$, NH-$(C_1-C_6)$-Alkyl, N($(C_1-C_6)$-Alkyl$)_2$, NH-CO-$(C_1-C_6)$-Alkyl, NH-COO-$(C_1-C_6)$-Alkyl, NH-CO-Aryl, NH-CO-Heterocyclus, NH-COO-Aryl, NH-COO-Heterocyclus, NH-CO-NH-$(C_1-C_6)$-Alkyl, NH-CO-NH-Aryl, NH-CO-NH-Heterocyclus, N($(C_1-C_6)$-Alkyl)-CO-$(C_1-C_6)$-Alkyl, N($(C_1-C_6)$-Alkyl)-COO-$(C_1-C_6)$-Alkyl, N($(C_1-C_6)$-Alkyl)-CO-Aryl, N($(C_1-C_6)$-Alkyl)-CO-Heterocyclus, N($(C_1-C_6)$-Alkyl)-COO-Aryl, N($(C_1-C_6)$-Alkyl)-COO-Heterocyclus, N($(C_1-C_6)$-Alkyl)-CO-NH-$(C_1-C_6)$-Alkyl), N($(C_1-C_6)$-Alkyl)-CO-NH-Aryl, N($(C_1-C_6)$-Alkyl)-CO-NH-Heterocyclus, N($(C_1-C_6)$-Alkyl)-CO-N($(C_1-C_6)$-Alkyl$)_2$, N($(C_1-C_6)$-Alkyl)-CO-N($(C_1-C_6)$-Alkyl)-Aryl, N($(C_1-C_6)$-Alkyl)-CO-N($(C_1-C_6)$-Alkyl)-Heterocyclus, N($(C_1-C_6)$-Alkyl)-CO-N-(Aryl$)_2$, N($(C_1-C_6)$-Alkyl)-CO-N-(Heterocyclus$)_2$, N (Aryl)-CO-$(C_1-C_6)$-Alkyl, N(Heterocyclus)-CO-$(C_1-C_6)$-Alkyl, N(Aryl)-COO-$(C_1-C_6)$-Alkyl, N(Heterocyclus)-COO-$(C_1-C_6)$-Akyl, N(Aryl)-CO-Aryl, N(Heterocyclus)-CO-Aryl, N(Aryl)-COO-Aryl, N(Heterocyclus)-COO-Aryl, N (Aryl)-CO-NH-$(C_1-C_6)$-Alkyl), N(Heterocyclus)-CO-NH-$(C_1-C_6)$-Alkyl), N(Aryl)-CO-NH-Aryl, N(Heterocyclus)-CO-NH-Aryl, N(Aryl)-CO-N($(C_1-C_6)$-Alkyl$)_2$, N(Heterocyclus)-CO-N($(C_1-C_6)$-Alkyl$)_2$, N(Aryl)-CO-N($(C_1-C_6)$-Alkyl)-Aryl, N(Heterocyclus)-CO-N($(C_1-C_6)$-Alkyl)-Aryl, N(Aryl)-CO-N-(Aryl$)_2$, N(Heterocyclus)-CO-N-(Aryl$)_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-$(C_1-C_6)$-Akyl, $(C_1-C_6)$-Alkyl, $NH_2$, NH$(C_1-C_6)$-Alkyl, N($(C_1-C_6)$-Akyl$)_2$, $SO_2$-$CH_3$, COOH, COO-$(C_1-C_6)$-Alkyl oder $CONH_2$.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Butinyl, Hexinyl.

Die Alkinylreste können ein oder mehrfach mit geeigneten Gruppen substituiert sein, wie z.B.: F, Cl, Br, I, $CF_3$, $NO_2$, $N_3$, CN, COOH, COO$(C_1-C_6)$Alkyl, $CONH_2$, CONH$(C_1-C_6)$Alkyl, CON[$(C_1-C_6)$Allcyl$]_2$, Cycloalkyl, $(C_2-C_6)$-Alkenyl, $(C_2-C_6)$-Alkinyl, O-$(C_1-C_6)$-Alkyl, O-CO-$(C_1-C_6)$-Alkyl, O-CO-$(C_1-C_6)$-Aryl, O-CO-$(C_1-C_6)$-Heterocyclus; $PO_3H_2$, $SO_3H$, $SO_2$-$NH_2$, $SO_2NH(C_1-C_6)$-Alkyl, $SO_2N[(C_1-C_6)$-Alkyl$]_2$, S-$(C_1-C_6)$-Alkyl, S-$(CH_2)_n$-Aryl, S-$(CH_2)_n$-Heteroeyelus, SO-$(C_1-C_6)$-Alkyl, SO-$(CH_2)_n$-Aryl, SO-$(CH_2)_n$-Heterocyclus, $SO_2$-$(C_1-C_6)$-Alkyl, $SO_2$-$(CH_2)_n$,-Aryl, $SO_2$-$(CH_2)_n$-Heterocyclus, $SO_2$-NH$(CH_2)_n$-Aryl, $SO_2$-NH$(CH_2)_n$-Heterocyclus, $SO_2$-N($(C_1-C_6)$-Alkyl)$(CH_2)_n$-Aryl, $SO_2$-N($(C_1-C_6)$-Alkyl) $(CH_2)_n$-Heterocyclus, $SO_2$-N($(CH_2)_n$-Aryl$)_2$, $SO_2$-N($(CH_2)_n$-Heterocyclus$)_2$ wobei n = 0 - 6 sein kann und der Arylrest oder Heterocyclische Rest bis zu zweifach mit F, Cl, Br, OH, $CF_3$, $NO_2$, CN, $OCF_3$, O-$(C_1-C_6)$-Alkyl, $(C_1-C_6)$-Alkyl oder $NH_2$ substituiert sein kann; C(=NH)($NH_2$), $NH_2$, NH-$(C_1-C_6)$-Alkyl, N($(C_1-C_6)$-Alkyl$)_2$, NH-CO-$(C_1-C_6)$-Alkyl, NH-

COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Akyl)-Aryl, N(Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-($CH_2$)$_n$-Aryl und O-($CH_2$)$_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Aryl, S-($CH_2$)$_n$-Heterocyclus, SO-($C_1$-$C_6$)-Alkyl, SO-($CH_2$)$_n$-Aryl, SO-($CH_2$)$_n$-Heterocyclus, $SO_2$-($C_1$-$C_6$)-Alkyl, $SO_2$-($CH_2$)$_n$-Aryl, $SO_2$-($CH_2$)$_n$-Heterocyclus, $SO_2$-NH($CH_2$)$_n$-Aryl, $SO_2$-NH($CH_2$)$_n$-Heterocyclus, $SO_2$-N(($C_1$-$C_6$)-Alkyl)($CH_2$)$_n$-Aryl, $SO_2$-N(($C_1$-$C_6$)-Alkyl)($CH_2$)$_n$-Heterocyclus, $SO_2$-N(($CH_2$)$_n$-Aryl)$_2$, $SO_2$-N(($CH_2$)$_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$ substituiert sein kann; C(=NH)($NH_2$), $NH_2$, NH-($C_1$-$C_6$)-Alkyl, N(($C_1$-$C_6$)-Alkyl)$_2$, NH-CO-($C_1$-$C_6$)-Alkyl, NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-($CH_2$)$_n$-Aryl und O-($CH_2$)$_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Akyl]$_2$ , S-($C_1$-$C_6$)-Alkyl, S-($CH_2$)$_n$-Aryl, S-($CH_2$)$_n$-Heterocyclus, SO-($C_1$-$C_6$)-Alkyl, SO-($CH_2$)$_n$-Aryl, SO-($CH_2$)$_n$-Heterocyclus, $SO_2$-($C_1$-$C_6$)-Alkyl, $SO_2$-($CH_2$)$_n$-Aryl, $SO_2$-($CH_2$)$_n$-Heterocyclus, $SO_2$-NH($CH_2$)$_n$-Aryl, $SO_2$-NH($CH_2$)$_n$-Heterocyclus, $SO_2$-N(($C_1$-$C_6$)-Alkyl)($CH_2$)$_n$-Aryl, $SO_2$-N(($C_1$-$C_6$)-Alkyl)($CH_2$)$_n$-Heterocyclus, $SO_2$-N(($CH_2$)$_n$-Aryl)$_2$, $SO_2$-N(($CH_2$)$_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$ substituiert sein kann; C(=NH)($NH_2$), $NH_2$, NH-($C_1$-$C_6$)-Alkyl, N(($C_1$-$C_6$)-Alkyl)$_2$, NH-CO-($C_1$-$C_6$)-Alkyl, NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N(Aryl)-COO-($C_1$-$C_6$)-Alkyl, N(Heterocy-

clus)-COO-($C_1$-$C_6$)-Alkyl, N(Aryl)-CO-Aryl, N(Heterocyclus)-CO-Aryl, N(Aryl)-COO-Aryl, N(Heterocyclus)-COO-Aryl, N (Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-$(CH_2)_n$-Aryl und O-$(CH_2)_n$-Heterocycluss wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Allyl) 2, $SO_2$-$CH_3$, COOH, COO-($C_1$-$C_6$)-Allcyl oder $CONH_2$.

Unter Heterocyclyl, Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit Benzolkernen kondensiert ist. Der Heterocyclus bzw. der heterocyclische Rest kann aromatisch, gesättigt aliphatisch oder teilweise ungesättigt aliphatisch sein.

Geeignete Heterocyclyl-Reste, bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterocyclen.

Die Heterocyclischen Ringe bzw. Heterocyclische Reste können ein oder mehrfach mit geeigneten Gruppen substituiert sein, wie z.B: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-$(CH_2)_n$-Aryl, S-$(CH_2)_n$-Heterocyclus, SO-($C_1$-$C_6$)-Alkyl, SO-$(CH_2)_n$-Aryl, SO-$(CH_2)_n$-Heterocyclus, $SO_2$-($C_1$-$C_6$)-Alkyl, $SO_2$-$(CH_2)_n$-Aryl, $SO_2$-($CH_2$)-Heteroeyclus, $SO_2$-NH$(CH_2)_n$-Aryl, $SO_2$-NH$(CH_2)_n$-Heterocyclus, $SO_2$-N(($C_1$-$C_6$)-Alkyl)($CH_2$)-Aryl,$SO_2$-N(($C_1$-$C_6$)-Alkyl)$(CH_2)_n$-Heterocyclus,$SO_2$-N(($CH_2$)-Aryl)$_2$,$SO_2$-N($(CH_2)_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$ substituiert sein kann; C(=NH)($NH_2$), $NH_2$, NH-($C_1$-$C_6$)-Alkyl N(($C_1$-$C_6$)-Alkyl)$_2$, NH-CO-($C_1$-$C_6$)-Alkyl, NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N (Aryl)-COO-($C_1$-$C_6$)-Alkyl, N(Heterocyclus)-COO-($C_1$-$C_6$)-Alkyl, N(Aryl)-CO-Aryl, N(Heterocyclus)-CO-Aryl, N (Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N (Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als

Infusion von 10 ng bis 100 ng pro Kilogramm pro Minute verabreicht werden kann. Geeignete Infusionslösungen für diese Zwecke können z.B. von 0,1 ng bis 10 ng, typischerweise von 1 ng bis 10 ng pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für

transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I können allein, aber oder auch in Kombination mit weiteren Wirkstoffen verabreicht werden. Als weitere Wirkstoffe für die Kombinationspräparate sind geeignet:

Alle Antidiabetika, die in der Roten Liste 2005, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder Apidra®, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulfonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken (PPAR = peroxisome proliferator activated receptor, PXR = pregnane X receptor, ATP = adenosine triphosphat).

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht (HMGCoA = 3-hydroxy-3-methylglutaryl Coenzyme A).

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, WO 2004/000804, WO 2004/000803, WO 2004/000805, EP 0114531, US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alfa Agonist, wie z.B. GW 9578, GW 7647, verabreicht.

Bei einer Ausführungsform der Erfindung werden die Verbindungen der Formel I in Kombination mit einem gemischten PPAR alfa/gamma Agonisten, wie z.B. GW 1536, AVE 8042, AVE 8134, AVE 0847, oder wie in WO 00/64888, WO 00/64876, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht (MTP = Microsomal triglyceride transfer protein).

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht (CETP = cholesteryl ester transfer protein).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. Her1171, HMR1586, verabreicht (LDL = Low Density Lipids).

Bei einer Ausführungsform der Erfindung werden die Verbindungen der Formel I in Kombination mit einem ACAT-hihibitor, wie z.B. Avasimibe, verabreicht (ACAT = acyl-Coenzyme A:cholesterol acyltransferase).

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulfonylharnstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausfülurungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylinethoxi]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Adenosin A1 Agonisten, wie z. B. solchen, die in der WO 2004/003002 beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausfiihrungsform werden die Verbindungen der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten (NPY = Neuropeptid Y, z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (MC4 = Melanocortin 4 receptor, z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (H3 = Histamin Rezeptor, z.B. 3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten (TNF = Tumor Necrosis Factor), CRF-Antagonisten (CRF = corticotropin releasing factor, z.B. [2-Methyl-9-(2,4,6-trimethylphenyl)-9H-1,3,9-triaza-fluoren-4-yl]-dipropyl-amin (WO 00/66585)), CRF BP-Antagonisten (CRF-BP = corticotropin releasing factor -binding protein, z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxi)-ethylamino]-ethanol Hydrochlorid (WO 01/83451)), CB1 (Cannabinoid Rezeptor 1) Rezeptor Antagonisten (zB. Rimonabant oder die in WO 02/28346 genannten Wirkstoffe, MSH (Melanocyt-stimulierendes Hormon)-Agonisten, CCK-A (CCK-A = cholecystokinin-A) Agonisten (z.B. {2-[4-(4-Chloro-2,5-dimethoxi-phenyl)-5-(2-cyclohexylethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten (Serotoninmimetika), z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxi-1-(2-diisopropylamino-ethylcarbamoyl)-3,4-dihydro-1H-isochinolin-2-carbonsäuretertiärbutylester (WO 01/85695)), TRH-Agonisten (TRH = TSH Releasing Hormone; TSH = thyroidstimulating hormone; thyrotropin ), siehe z.B. EP 0 462 884 entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (DA = Dopamine Autoreceptor, wie z.B. Bromocriptin, Doprexin), Lipase/Amylase-Inhibitoren (z.B. WO 00/40569), PPAR-Modulatoren (z.B. WO 00/78312), RXR- (RXR = Retinoid X Receptor) Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.); Caromax ist ein Carob enthaltendes Produkt der Fa.

Nutrinova, Nutrition Specialties & Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

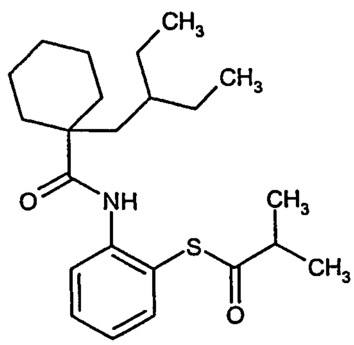

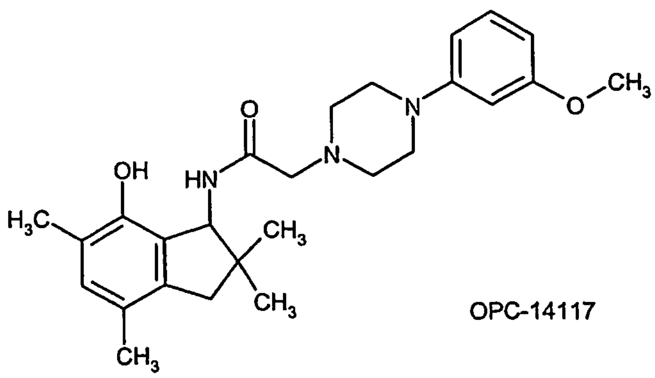

OPC-14117

JTT-705

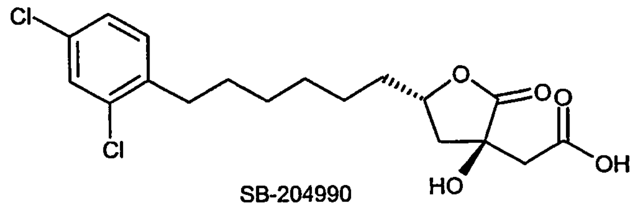

SB-204990

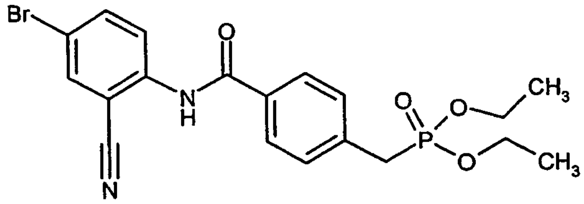

NO-1886

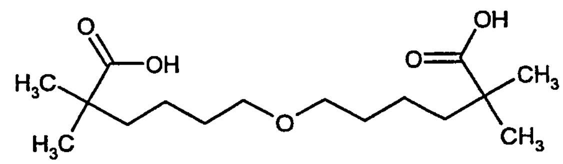

CI-1027

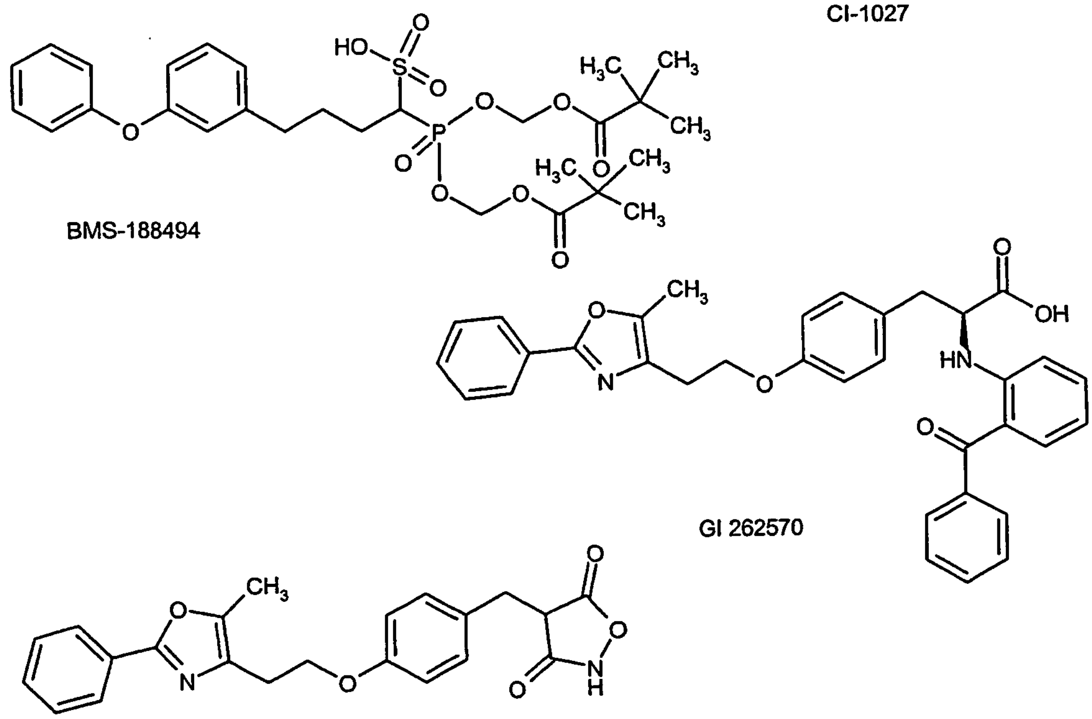

BMS-188494

GI 262570

JTT-501

Die Verbindungen der Formel I lassen sich dadurch herstellen, dass man geeignete Ausgangsstoffe der Formel II, worin X eine Austrittsgruppe, wie Chlor, Brom, Jod, Sulfonyloxi, Sulfinyl oder Sulfoxyl, bedeutet, mit einer Verbindung der Formel IV ggf. in Gegenwart von geeigneten Basen und in geeigneten Lösemitteln umsetzt.

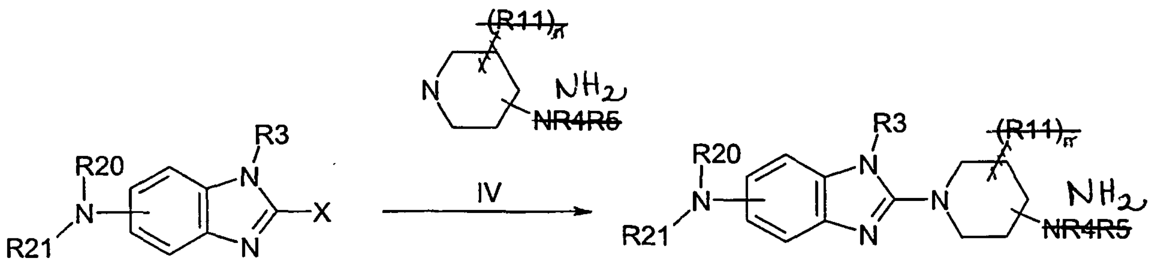


II

I

Es kann zweckmäßig sein, den Rest IV in an der Stickstofffunktion geschützter Form einzusetzen und die Schutzgruppe nach erfolgter Reaktion mit II wieder abzuspalten. Solche geeignete Schutzgruppen und die Verfahren der Einführung und Abspaltung sind bekannt (Siehe:Theodora W. Greene and Peter G. M. Wuts, Protective Groups in Organic Synthesis, 3rd Edition, John Wiley & Sons, Inc., New York, 1999)

Die Halogenverbindungen der Formel II können nach bekannten Verfahren wie beispielsweise durch Halogenierung der entsprechenden H-, Hydroxy- oder Thio-Verbindung (Formel II, X = H, OH oder SH) erhalten werden. Geeignete Halogenierungsmittel können beispielhaft Halogene, wie Chlor und Brom, N-Bromsuccinimid, Phosphorpentachlorid oder Phosphoroxychlorid sein.

Die Synthese von Verbindungen der Formel II ist in der Literatur beschrieben. Sie können beispielsweise durch Kondensation von substituierten Diaminobenzolderivaten mit Aldehyden in Gegenwart eines Oxidationsmitteln (z.B. Luftsauerstoff, Sauerstoff, Iod, Oxone, Chinonen, Peroxiden, usw.) oder alternativ mit Carbonsäuren, Nitrilen oder Amiden ohne oder in Gegenwart eines Katalysators hergestellt werden.

Die Synthese der Amine IV kann nach literaturbekannten Verfahren erfolgen.

Einige Derivate der Formel IV, wie zum Beispiel Piperidin-3-ylamine, sind kommerziell erhältlich.

Die nachfolgend aufgeführten tabellarischen Beispiele dienen zur Erläuterung der Erfindung, ohne diese jedoch einzuschränken.

Tabelle 1:

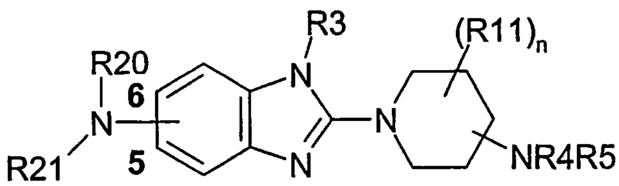

I

| Bsp. | Position von -NR20R21 | R20 | R21 | R3 | NR4R5 | n | R11 |
|---|---|---|---|---|---|---|---|
| 1 | 6 | H | Phenyl-$SO_2$- | -$CH_2$-CH=C $(CH_3)_2$ | 3-R-$NH_2$ x TFA | 0 | - |
| 2 | 6 | Phenyl-$SO_2$- | Phenyl-$SO_2$- | Benzyl- | 3-R-$NH_2$ x TFA | 0 | - |
| 3 | 6 | H | 2-Cl-4-F-Benzyl- | Benzyl- | 3-R-$NH_2$ x TFA | 0 | - |
| 4 | 6 | Benzyl- | Benzyl- | Benzyl- | 3-R-$NH_2$ x TFA | 0 | - |

(fortgesetzt)

| Bsp. | Position von -NR20R21 | R20 | R21 | R3 | NR4R5 | n | R11 |
|---|---|---|---|---|---|---|---|
| 5 | 6 | 2-Cl-4-F-Benzyl- | 2-Cl-4-F-Benzyl- | Benzyl- | 3-R-$NH_2$ x TFA | 0 | - |
| 6 | 6 | H | Benzyl- | $-CH_2-CH=C(CH_3)_2$ | 3-R-$NH_2$ x TFA | 0 | - |
| 7 | 6 | H | H | $-CH_2-CH=C(CH_3)_2$ | 3-R-$NH_2$ x TFA | 0 | - |
| 8 | 6 | H | H | Benzyl- | 3-R-$NH_2$ x TFA | 0 | - |
| 9 | 5 | H | H | $-CH_2-CH=C(CH_3)_2$ | 3-R-$NH_2$ x TFA | 0 | - |

Die Verbindungen der Formel I zeichnen sich durch günstige Wirkungen auf den Lipid- und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV (Dipeptidyl Peptidase IV) Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen und zur Behandlung von Drogenmissbrauch.
Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:

Messung der DPP-IV Aktivität:

Material:

DPP-IV aus Schweineniere (Sigma, München)

H-Ala-Pro-AFC (Bachem, Weil am Rhein)

Testbedingungen:

DPP-IV (1 mU/ml, Endkonzentration)

H-Ala-Pro-AFC (15 $\mu$M Endkonzentration)
in Tris/HCl (40 mM, pH 7.4), Gesamtvolumen 0,2 ml

Die Reaktion wurde bei Raumtemperatur für unterschiedliche Zeiträume (typischerweise 10 Minuten) durchgeführt und am Ende der Reaktion durch Zugabe von 20 $\mu$l $ZnCl_2$ (1 M) gestoppt. Der Umsatz von H-Ala-Pro-AFC wurde fluorimetrisch durch Messung der Emission bei 535 nm nach Anregung bei 405 nm bestimmt. Im Falle der Zugabe von Inhibitoren wurde das zugegebene Puffervolumen so angepasst, dass ein Gesamtvolumen der Testmischung von 200 $\mu$l eingehalten wurde.

%Hemmung bei einer festen Konzentration wurden wie folgt errechnet:

$$(1\text{-Enzymaktivität}_{\text{gehemmte Reaktion}} / \text{Enzymaktivität}_{\text{ungehemmte Reaktion}}) \times 100$$

$IC_{50}$ Werte für Inhibitoren wurden durch Variation der Inhibitorkonzentrationen bei der angegebenen Substratkonzentration von 15 µM bestimmt. $K_i$ und $K_m$ Werte wurden durch enstsprechende Variation von Substrat- und Inhibitorkonzentration wie beschrieben (Dixon, M. and Webb, E.C.(1979) Enzymes , third edition, pp. 47-206, Academic Press) ermittelt. Die Werte für $K_m$, $IC_{50}$ and $K_i$ wurden mit einem kommerziell erhältlichen Software-Paket errechnet (Leatherbarrow, R.J. (1992) GraFit Version 3.0, Erithacus Software Ltd. Staines, U.K.).

Tabelle 2: Biologische Aktivität der Beispiele:

| Beispiel | % Hemmung bei 30 µM |
|---|---|
| 1 | 56 |
| 2 | 8 |
| 4 | 75 |
| 6 | 73 |
| 9 | 34 |

Aus der Tabelle ist abzulesen, dass die Verbindungen der Formel I die Aktivität der DPP-IV (Dipeptidyl Peptidase IV) hemmen und dadurch zur Senkung des Blutzuckerspiegels geeignet sind.

Nachfolgend wird die Herstellung einiger Ausführungsbeispiele detailliert beschrieben, die übrigen Verbindungen der Formel I wurden analog erhalten:

Beispiel 1

R-N-[2-(3-R-Amino-piperidin-1-yl)-1-(3-methyl-but-2-enyl)-1H-benzimidazol-6-yl]-benzolsulfonamid Trifluoressigsäuresalz

a) 2-Brom-5/6-nitro-1H-benzimidazol

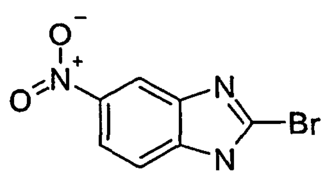

Eine Suspension von 5,0 g (25,61 mmol) 5/6-Nitro-1H-benzimidazol-2-thiol in 30 ml Methanol und 10 ml Bromwasserstoff (48%ig in Wasser) wurde auf 5-10°C abgekühlt und mit 3,55 g (22,2 mmol) Brom versetzt. Anschließend wurde 45 Minuten bei 5-10°C gerührt und mit 8 ml Methanol/wässrige $NH_3$-Lösung = 3/1 versetzt. Der Niederschlag wurde abgesaugt und die Mutterlauge auf Eiswasser gegossen. Dabei entstand erneut ein Niederschalg, der ebenfalls abgesaugt wurde. Die vereinigten Niederschläge wurden zwischen Essigsäureethylester und Wasser verteilt, getrocknet und im Vakuum eingeengt. Man erhielt 1,12 g des gewünschten Produktes, das ohne weitere Reinigung in der nächsten Stufe eingesetzt wurde.

b) 2-Brom-1-(3-methyl-but-2-enyl)-5/6-nitro-1H-benzimidazol

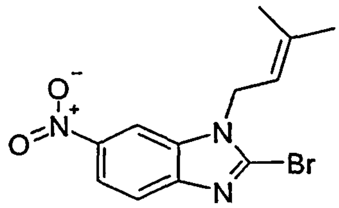

0,55 g (2,27 mmol) 2-Brom-5/6-nitro-1H-benzimidazol wurden in 10 ml Dimethylformamid gelöst, mit 1,11 g (3,41 mmol) Cäsiumkarbonat versetzt und 30 Minuten bei Raumtemperatur gerührt. 408 mg (2,50 mmol) 1-Brom-3-methyl-2-buten wurden zugegeben und das Reaktionsgemisch wurde 4 Stunden bei Raumtemperatur gerührt. Der Niederschlag wurde abgesaugt und mit Dimethylformamid gewaschen. Das Filtrat wurde im Vakuum eingeengt und der Rückstand zwischen Essigsäureethylester und Wasser verteilt. Die organische Phase wurde getrocknet und im Vakuum eingeengt. Man erhielt 371 mg (53%) des gewünschten Produktes. LC/MS: m/z = 310,0/312,0 $(M+H)^+$.

c) R-{1-[1-(3-Methyl-but-2-enyl)-6-nitro-1H-benzimidazol-2-yl]-piperidin-3-yl}-carbaminsäure-tert-butylester

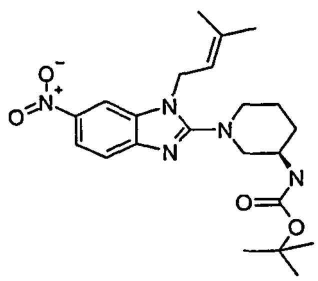

260 mg (1,29 mmol) R-Piperidin-3-yl-carbaminsäure-tert-butylester wurden in 2 ml Dimethylformamid gelöst und mit 575 mg (1,77 mmol) Cäsiumkarbonat versetzt und 30 Minuten bei Raumtemperatur gerührt. 365 mg (1,18 mmol) 2-Brom-1-(3-methyl-but-2-enyl)-5/6-nitro-1H-benzimidazol wurden in 8 ml Dimethylformamid gelöst und langsam zugegeben. Das Reaktionsgemisch wurde 6 Stunden bei 40°C gerührt. Der Niederschlag wurde abgesaugt und mit Dimethylformamid gewaschen. Das Filtrat wurde eingeengt und zwischen Essigsäueethylester und Wasser verteilt. Die organische Phase wurde getrocknet und im Vakuum eingeengt. Das Rohgemisch wurde an Kieselgel getrennt (Laufmittel: Heptan/Essigsäureethylester, Gradient: 3/1 auf 1/1). Man erhielt 176 mg (35%) R-{1-[1-(3-Methyl-but-2-enyl)-5-nitro-1H-benzimidazol-2-yl]-piperidin-3-yl}-carbaminsäure-tert-butylester und 163 mg (32%) des gewünschten Produktes. LC/MS: m/z = 430,2 $(M+H)^+$.

d) R-{1-[6-Amino-1-(3-methyl-but-2-enyl)-1H-benzimidazol-2-yl]-piperidin-3-yl}-carbaminsäure-tert-butylester

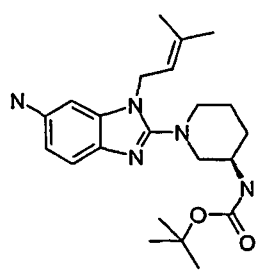

Zu einer Suspension von 106 mg (1,90 mmol) Eisen und 18 mg (0,34 mmol) Ammoniumchlorid in 1 ml Wasser wurde eine Lösung von 163 mg (0,38 mmol) R-{1-[1-(3-Methyl-but-2-enyl)-6-nitro-1H-benzimidazol-2-yl]-piperidin-3-yl}-carbaminsäure-tert-butylester in 10 ml Ethanol getropft und 3 Stunden bei Rückfluss gekocht. Der Katalysator wurde abfiltriert und mit Ethanol gewaschen. Das Filtrat wurde im Vakuum eingeengt. Man erhielt 155 mg des gewünschten Produktes, das ohne weitere Reinigung in der nächsten Stufe umgesetzt wurde.
MS: m/z = 400,3 $(M+H)^+$.

e) R-{1-[6-Benzolsulfonylamino-1-(3-methyl-but-2-enyl)-1H-benzimidazol-2-yl]-piperidin-3-yl}-carbaminsäure-tert-butylester

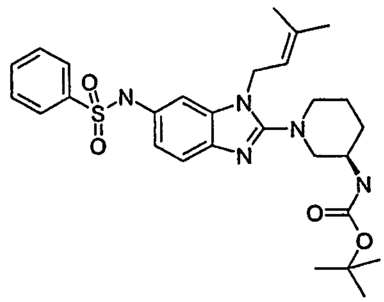

Zu einer Lösung von 50 mg (0,13 mmol) R-{1-[6-Amino-1-(3-methyl-but-2-enyl)-1H-benzimidazol-2-yl]-piperidin-3-yl}-carbaminsäure-tert-butylester in 5 ml Dimethylformamid gab man 20 mg (0,06 mmol) Cäsiumkarbonat und rührte 30 Minuten bei Raumtemperatur. Anschließend wurden 22 µl (0,13 mmol) Benzolsulfonylchlorid zugegeben und 20 Stunden bei Raumtemperatur gerührt. Das Reaktionsgemisch wurde zwischen Dichlormethan und Wasser verteilt, die organische Phase wurde getrocknet und im Vakuum eingeengt. Das Rohgemisch wurde an Kieselgel getrennt (Laufmittel: Heptan/Essigsäureethylester, Gradient: 1/1 auf 0/1). Man erhielt 43 mg (63%) der gewünschten Verbindung.
MS: m/z = 540,5 $(M+H)^+$.

f) R-N-[2-(3-Amino-piperidin-1-yl)-1-(3-methyl-but-2-enyl)-1H-benzimidazol-6-yl]-benzolsulfonamid Trifluoressigsäuresalz

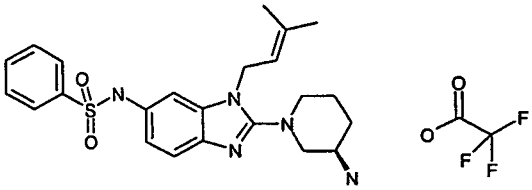

43 mg (0,08 mmol) R-{1-[6-Benzolsulfonylamino-1-(3-methyl-but-2-enyl)-1H-benzimidazol-2-yl]-piperidin-3-yl}-carbaminsäure-tert-butylester wurden in 182 mg Trifluoressigsäure und 12 µl Wasser gelöst und 19 Stunden bei Raumtemperatur gerührt. Das Reaktionsgemisch wurde mit Wasser versetzt und gefriergetrocknet. Man erhielt 48 mg des gewünschten Produktes in quantitativer Ausbeute.
MS: m/z = 440,4 $(M+H)^+$.

## Patentansprüche

**1.** Verwendung der Verbindungen der Formel I,

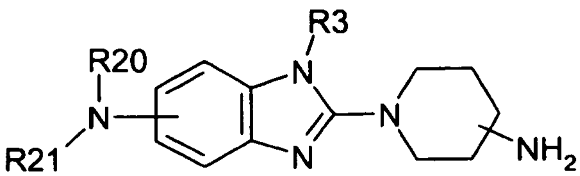

I

worin bedeuten

R20, R21 unabhängig voneinander H, $(C_1-C_6)$-Alkylen-$(C_6-C_{10})$-Aryl oder $S(O)_2$-Aryl, wobei die Aryl-Reste ein- oder mehrfach substituiert sein können mit F, Cl, Br, I, CN, $NO_2$, SH, OH, $(C_1-C_6)$-Alkyl, O-$(C_1-C_6)$-Alkyl oder S-$(C_1-C_6)$-Alkyl;
R3 $(C_2-C_{10})$-Alkenyl, $(C_1-C_6)$-Alkylen-$(C_6-C_{10})$-Aryl;

sowie derer physiologisch verträglichen Salze zur Herstellung eines Medikaments zur Blutzuckersenkung

2. Verwendung der Verbindungen der Formel I, gemäß Anspruch 1, sowie derer physiologisch verträglichen Salze zur Herstellung eines Medikaments zur Behandlung von Diabetes.

## Claims

1. The use of a compound of the formula I

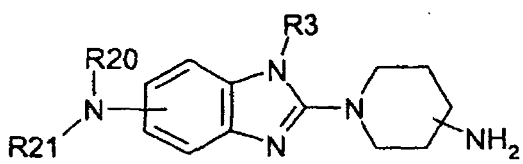

I

in which

R20, R21 are each independently H, $(C_1-C_6)$-alkylene-$(C_6-C_{10})$-aryl or $S(O)_2$-aryl, where the aryl radicals may each be mono- or polysubstituted by F, Cl, Br, I, CN, $NO_2$, SH, OH, $(C_1-C_6)$-alkyl, O-$(C_1-C_6)$-alkyl or S-$(C_1-C_6)$-alkyl;
R3 is $(C_2-C_{10})$-alkenyl, $(C_1-C_6)$-alkylene-$(C_6-C_{10})$-aryl;

and physiologically compatible salts thereof for producing a medicament for lowering blood sugar.

2. The use of the compounds of the formula I as claimed in claim 1, and physiologically compatible salts thereof for producing a medicament for treating diabetes.

## Revendications

1. Utilisation des composés de formule I,

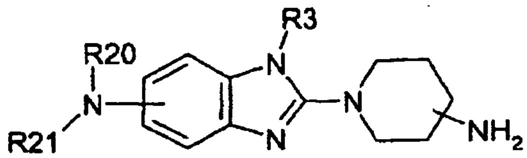

I

dans laquelle

R20, R21 signifient, indépendamment l'un de l'autre, H, $(C_1-C_6)$-alkylène-$(C_6-C_{10})$-aryle ou $S(O)_2$-aryle, où les radicaux alkyle peuvent être monosubstitués ou polysubstitués par F, Cl, Br, I, CN, $NO_2$, SH, OH, $(C_1-C_6)$-alkyle, O-$(C_1-C_6)$-alkyle ou S-$(C_1-C_6)$-alkyle ;
R3 signifie $(C_2-C_{10})$-alcényle, $(C_1-C_6)$ -alkylène- $(C_6-C_{10})$-aryle ;

ainsi que leurs sels physiologiquement acceptables pour la préparation d'un médicament destiné à la diminution de la glycémie.

**2.** Utilisation des composés de formule I selon la revendication 1 ainsi que de leurs sels physiologiquement acceptables pour la préparation d'un médicament destiné au traitement du diabète.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1069124 A
- WO 9712615 A
- WO 0204425 A
- US 6221633 B
- WO 9808871 A
- WO 9726265 A
- WO 9903861 A
- EP 200400269 W
- WO 2004000804 A
- WO 2004000803 A
- WO 2004000805 A
- EP 0114531 A
- US 6498156 B
- WO 0064888 A
- WO 0064876 A
- DE 10142734
- US 6245744 B
- US 6221897 B
- US 6342512 B
- WO 9741097 A
- WO 2004003002 A
- WO 0191752 A
- WO 0063208 A
- WO 0066585 A
- WO 0183451 A
- WO 0228346 A
- WO 9915525 A
- WO 0071549 A
- WO 0109111 A
- WO 0185695 A
- EP 0462884 A
- WO 0040569 A
- WO 0078312 A


### In der Beschreibung aufgeführte Nicht-Patentliteratur


- *Pharmaceutical Research,* 1986, vol. 2 (6), 318
- **Asakawa, A et al.** *M.: Hormone and Metabolic Research,* 2001, vol. 33 (9), 554-558
- **Lee, Daniel W. ; Leinung, Matthew C. ; Rozhavskaya-Arena, Marina ; Grasso, Patricia.** Leptin agonists as a potential approach to the treatment of obesity. *Drugs of the Future,* 2001, vol. 26 (9), 873-881
- **Salvador, Javier ; Gomez-Ambrosi, Javier ; Fruhbeck, Gema.** Perspectives in the therapeutic use of leptin. *Expert Opinion on Pharmacotherapy,* 2001, vol. 2 (10), 1615-1622
- **Zunft H J et al.** Carob pulp preparation for treatment of hypercholesterolemia. *ADVANCES IN THERAPY,* September 2001, vol. 18 (5), 230-6
- **Theodora W. Greene ; Peter G. M. Wuts.** Protective Groups in Organic Synthesis. John Wiley & Sons, Inc, 1999
- **Dixon, M. ; Webb, E.C.** Enzymes. Academic Press, 1979, 47-206
- **Leatherbarrow, R.J.** GraFit. Erithacus Software Ltd. Staines, 1992